# EUROPÄISCHE PATENTANMELDUNG

(11) **EP 1 774 987 A1**
(43) Veröffentlichungstag der Anmeldung: **18.04.2007**
(21) Anmeldenummer: 05022225.6
(22) Anmeldetag: 12.10.2005
(51) Int. Cl.: A61N 1/32, A61N 1/34

(54) **Elektrode zur Nerven- und/oder Muskelstimulation und Verfahren zur Nerven- und/oder Muskelstimulation**

(71) Anmelder: Pierenkemper GmbH, 35578 Wetzlar (DE)
(72) Erfinder: Kreutner, Bernd-Jürgen, 35630 Ehringshausen (DE)
(74) Vertreter: Knefel, Cordula

(57) **Zusammenfassung**

Die Erfindung betrifft eine Elektrode zur Nerven- und/oder Muskelstimulation, die auf der Haut eines Patienten anordbar ist, wobei die Elektrode als eine wenigstens ein Medikament aufweisende Elektrode ausgebildet ist, und wobei das wenigstens eine Medikament oder ein das wenigstens eine Medikament aufweisender Träger nach Anordnen der Elektrode auf der Haut als ein an der Haut anliegendes Medikament oder Träger ausgebildet ist. Die Erfindung betrifft darüber hinaus ein Verfahren zur Nerven- und/oder Muskelstimulation.

## Beschreibung

Die Erfindung betrifft eine Elektrode zur Nerven- und/oder Muskelstimulation sowie ein Verfahren zur Nerven- und/oder Muskelstimulation.

Gemäß dem Stand der Technik ist es bekannt, zur Schmerzbehandlung eine Nervenstimulation, beispielsweise eine transkutane elektrische Nervenstimulation (TENS) durchzuführen. Bei diesem zum Stand der Technik gehörenden Verfahren erzeugen Reizstromgeräte annähernd Rechteckimpulse bei einer Impulsdauer von circa 50 Mikrosekunden bis 500 Mikrosekunden, die in ihrer Intensität einstellbar sind. Die Frequenz der Rechteckimpulse liegt beispielsweise im Bereich von 0,5 Hertz bis 120 Hertz. Für eine Schmerzbehandlung werden Elektroden an den betreffenden Stellen des Körpers angeordnet und mit diesen Stromimpulsen beaufschlagt.

Die bei der Elektrotherapie verwendeten Elektroden sind beispielsweise als Selbstklebeelektroden ausgebildet, die eine leitfähige und klebefähige Masse aufweisen. Lässt die Klebefähigkeit nach, kann beispielsweise etwas Wasser auf die Oberfläche gegeben werden, um die Klebefähigkeit wieder herzustellen.

Die Elektroden weisen einen Anschluss auf, um sie an dem Gerät zur Elektrostimulation anzuschließen.

Weiterhin ist gemäß dem Stand der Technik ein Verfahren zum Einbringen von Medikamenten bekannt, und zwar die so genannte Iontophorese. Bei der Iontophorese werden elektrisch geladene, dissoziierte Wirkstoffe mit Hilfe des Stromes in die Haut transportiert. Es gibt zwei Gruppen von elektrisch geladenen Pharmaka, und zwar anionische und kationische. Bei der Iontophorese wird das Medikament, welches hierbei meist in einer Creme oder Salbe enthalten ist, direkt auf die Haut aufgetragen. Die Elektrode wird, da sie an der Creme oder Salbe nicht haftet, mit einem zusätzlichen Pflaster an der Haut fixiert. Zwischen die Haut beziehungsweise Creme und die Metallelektrode muss ein Schwämmchen oder Vlies gelegt werden, das mit handwarmem Leitungswasser getränkt wird, da die metallische Elektrode nicht unmittelbar mit der Creme in Kontakt kommen darf.

Dieses zum Stand der Technik gehörende Verfahren der Iontophorese hat den Nachteil, dass es relativ aufwändig und kompliziert ist, da zuerst die das Medikament enthaltende Creme oder Salbe aufgetragen werden muss. Anschließend muss die Elektrode im Bereich der aufgetragenen Creme oder Salbe platziert und mit einem Pflaster fixiert werden.

Das der Erfindung zugrunde liegende technische Problem besteht darin, eine Elektrode anzugeben, die eine einfache Anwendung der Iontophorese und eine Nerven- und/oder Muskelstimulation erlaubt. Darüber hinaus soll ein Verfahren angegeben werden, mit dem auf einfache Art und Weise die Durchführung einer Iontophorese mit einer zusätzlichen Nerven- und/oder Muskelstimulation möglich ist.

Dieses technische Problem wird durch eine Elektrode mit den Merkmalen gemäß Anspruch 1 sowie durch ein Verfahren mit den Merkmalen gemäß Anspruch 11 gelöst.

Die erfindungsgemäße Elektrode zur Nerven- und/oder Muskelstimulation zeichnet sich dadurch aus, dass sie auf der Haut eines Patienten anordbar ist, und dass die Elektrode als eine wenigstens ein Medikament aufweisende Elektrode ausgebildet ist.

Vorteilhaft ist das wenigstens eine Medikament oder ein das wenigstens eine Medikament aufweisender Träger nach Anordnen der Elektrode auf der Haut als ein an der Haut anliegendes Medikament oder Träger ausgebildet.

Hierdurch kommt das Medikament unmittelbar mit der Haut in Kontakt. Wird an die Elektrode ein monophasischer Strom angelegt, so wird das Medikament durch die Haut in die Blutbahn und das Gewebe eingebracht. Das Medikament wird durch die angelegte Spannung entweder negativ oder positiv aufgeladen. Mit der Iontophorese lassen sich direkt zugängliche Organe, wie Haut oder zum Beispiel darunter liegende Gewebe, wie die Gelenkinnenhaut, mit hohen Medikamentenkonzentrationen behandeln.

Die erfindungsgemäße Elektrode weist den Vorteil auf, dass sie Träger für das Medikament ist, so dass der Anwender die Elektrode lediglich auf der Haut anordnen muss.

Gemäß einer besonders bevorzugten Ausführungsform der Erfindung ist es möglich, monophasische, getaktete Stromimpulse durch die Elektrode zu leiten und hierdurch zusätzlich zu der Iontophorese eine Nerven- und/oder Muskelstimulation zu erreichen. Hierdurch wird besonders vorteilhaft eine sehr effektive Schmerztherapie möglich, da zum einen das Medikament durch die Iontophorese deutlich schneller in die Haut eindringt, als dies bei einem normalen Auftragen des Medikamentes auf die Haut geschieht, und die bekannten Wirkungen der Nerven- und Muskelstimulation, die in der Schmerztherapie gemäß dem Stand der Technik schon eingesetzt werden, treten ebenfalls ein.

Gemäß einer besonders vorteilhaften Ausführungsform der Erfindung ist die Elektrode als TENS-Elektrode (transkutane elektrische Nervenstimulation) ausgebildet. Mit diesen Elektroden lässt sich gleichzeitig die Iontophorese und die transkutane elektrische Nervenstimulation durchführen.

Gemäß einer weiteren besonders bevorzugten Ausführungsform der Erfindung ist die Elektrode als selbstklebende Elektrode ausgebildet, um die Handhabung mit der Elektrode für den Patienten zu vereinfachen.

Als Klebemittel ist vorteilhaft an der Elektrode ein leitfähiges Gel vorgesehen. Dieses Gel bewirkt die selbstklebende oder selbsthaftende Wirkung. Durch das Gel wird der Strom oder werden die Stromimpulse geleitet.

Gemäß einer besonders bevorzugten Ausführungsform der Erfindung ist das Gel als Träger für das wenigstens eine Medikament ausgebildet. Da das Gel ohnehin in unmittelbaren Kontakt mit der Haut gelangt, ist es vorteilhaft, das Gel direkt als Träger für das wenigstens eine Medikament zu verwenden.

Hierbei ist es besonders vorteilhaft, ein Gel auf Wasserbasis vorzusehen, um die Wirkung der Medikamente nicht zu beeinflussen.

Gemäß einer weiteren vorteilhaften Ausführungsform ist dem Gel wenigstens ein Wirkstoff des wenigstens einen Medikamentes beigemischt, so dass mit der erfindungsgemäßen Elektrode gleichzeitig die Iontophorese und die transkutane elektrische Nervenstimulation durchgeführt werden kann.

Vorteilhaft ist die erfindungsgemäße Elektrode als eine vorgefertigte Elektrode ausgebildet. Das bedeutet, dass verschiedene Elektroden mit verschiedenen Medikamenten im Handel käuflich erworben werden können. Der Arzt oder Patient entscheidet, welche Elektrode im Einzelfall einzusetzen ist. Hierbei kann zwischen verschiedenen Elektroden mit verschiedenen Medikamenten ausgewählt werden. Die Nerven- und/oder Muskelstimulation kann ebenfalls frei gewählt werden, je nach Anwendungsfall.

Die erfindungsgemäße Elektrode ist vorteilhaft mit einem monophasischen, getakteten Stromimpuls beaufschlagbar. Durch den monophasischen Strom ist gewährleistet, dass die Iontophorese stattfindet. Durch die Stromimpulse ist gewährleistet, dass eine Nerven- und/oder Muskelstimulation stattfindet.

Die Elektrode kann auch mehrschichtig ausgebildet sein. Es ist beispielsweise möglich, eine eigenstabile Schicht aus Gel vorzusehen, die das wenigstens eine Medikament enthält. Daran anschließen kann sich die Schicht aus dem selbsthaftenden Gel, mit der die Elektrode auf der Haut befestigt wird. Die Schicht mit dem selbsthaftenden Gel muss dann natürlich leitfähig für das Medikament ausgebildet sein. Voraussetzung ist dann natürlich auch, dass die Schicht, die als Trägerschicht für das wenigstens eine Medikament dient, elektrisch leitfähig ausgebildet ist.

Bei dem erfindungsgemäßen Verfahren wird die erfindungsgemäße Elektrode auf der Haut angeordnet und mit monophasischen Stromimpulsen beaufschlagt. Dadurch, dass die Elektrode das Medikament unmittelbar aufweist, ist eine leichte Handhabung möglich. Darüber hinaus ist das Medikament dosiert in der Elektrode angeordnet, was den Vorteil hat, dass beispielsweise nicht zu viel oder zu wenig Salbe aufgetragen wird.

Weitere Merkmale und Vorteile der Erfindung ergeben sich anhand der zugehörigen Zeichnung, in der ein Ausführungsbeispiel einer erfindungsgemäßen Elektrode nur beispielhaft dargestellt ist. In der Zeichnung zeigen:
- Fig. 1: eine Elektrode in Ansicht von unten;
- Fig. 2: einen Schnitt nach der Linie I-I der Fig. 1.

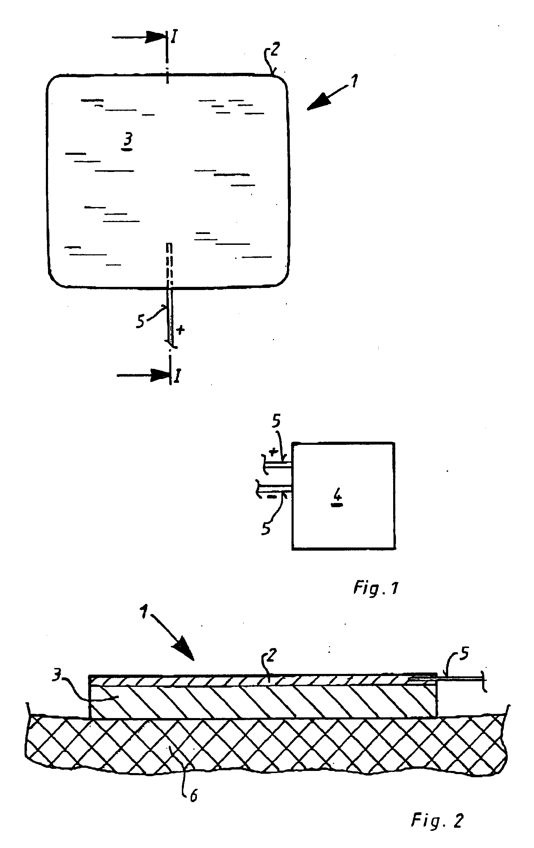

Die Fig. 1 und 2 zeigen eine selbstklebende Elektrode 1, die ein Trägermaterial 2 aufweist, auf dem eine leitfähige und klebefähige Masse 3, beispielsweise in Form eines Gels, angeordnet ist. Von einem Elektrostimulationsgerät 4 werden monophasische, getaktete Stromimpulse durch Leitungen 5 der Elektrode 1 zugeführt.

Wie der Fig. 2 zu entnehmen ist, ist die Elektrode 1 auf der Haut 6 aufgeklebt. Die Schicht 3 aus Gel steht in unmittelbarem Kontakt mit der Haut 6. In dem Gel 3 ist wenigstens ein Medikamentenwirkstoff angeordnet, das heißt, der Medikamentenwirkstoff ist dem Gel 3 beigemischt. Das Gel stellt eine leitfähige und klebefähige Masse dar, so dass das Gel 3 zum einen Träger für den Medikamentenwirkstoff ist und zum anderen die selbstklebende Eigenschaft der Elektrode 1 gewährleistet und darüber hinaus leitfähig ausgebildet ist, um die Stromimpulse auf die Haut 6 weiterzuleiten.

Durch die erfindungsgemäße Elektrode 1 ist es möglich, eine Nerven- und/oder Muskelstimulation durchzuführen und gleichzeitig durch Iontophorese den Medikamentenwirkstoff, der im Gel 3 angeordnet ist, in die Haut 6 einzubringen.

### Bezugszahlen

- 1: Elektrode
- 2: Trägermaterial
- 3: Gel
- 4: Elektrostimulationsgerät
- 5: Leitung
- 6: Haut

## Patentansprüche

1. Elektrode zur Nerven- und/oder Muskelstimulation, die auf Haut eines Patienten anordbar ist,
**dadurch gekennzeichnet, dass** die Elektrode (1) als eine wenigstens ein Medikament aufweisende Elektrode (1) ausgebildet ist.

2. Elektrode nach Anspruch 1, **dadurch gekennzeichnet, dass** das wenigstens eine Medikament oder ein das wenigstens eine Medikament aufweisender Träger (3) nach Anordnen der Elektrode (1) auf der Haut (6) als ein an der Haut (6) anliegendes Medikament oder Träger (3) ausgebildet ist.

3. Elektrode nach Anspruch 1, **dadurch gekennzeichnet, dass** die Elektrode (1) als eine für eine transkutane elektrische Nervenstimulation (TENS) einsetzbare Elektrode (1) ausgebildet ist.

4. Elektrode nach einem der Ansprüche 1 bis 3, **dadurch gekennzeichnet, dass** die Elektrode (1) als selbstklebende Elektrode (1) ausgebildet ist.

5. Elektrode nach Anspruch 4, **dadurch gekennzeichnet, dass** als Klebemittel ein an der Elektrode (1) angeordnetes elektrisch leitfähiges Gel (3) vorgesehen ist.

6. Elektrode nach Anspruch 5, **dadurch gekennzeichnet, dass** das Gel (3) als Träger für das wenigstens eine Medikament ausgebildet ist.

7. Elektrode nach Anspruch 5 oder 6, **dadurch gekennzeichnet, dass** ein Gel (3) auf Wasserbasis vorgesehen ist.

8. Elektrode nach Anspruch 5 oder 6, **dadurch gekennzeichnet, dass** dem Gel (3) wenigstens ein Wirkstoff des wenigstens einen Medikamentes beigemischt ist.

9. Elektrode nach Anspruch 1, **dadurch gekennzeichnet, dass** die das wenigstens eine Medikament aufweisende Elektrode (1) als eine vorgefertigte Elektrode (1) ausgebildet ist.

10. Elektrode nach Anspruch 4 oder 5, **dadurch gekennzeichnet, dass** die Elektrode (1) als vollflächig klebende Elektrode (1) ausgebildet ist.

11. Elektrode nach Anspruch 1, **dadurch gekennzeichnet, dass** die Elektrode (1) mit einem monophasischen, getakteten Stromimpuls beaufschlagbar ist.

12. Elektrode nach Anspruch 1, **dadurch gekennzeichnet, dass** die Elektrode einen mehrschichtigen Aufbau mit Gel und Trägerschicht mit dem wenigstens einen Medikament aufweist.

13. Verfahren zur Nerven- und/oder Muskelstimulation mit einer Elektrode nach einem der vorhergehenden Ansprüche, **dadurch gekennzeichnet, dass** die das Medikament aufweisende Elektrode (1) auf der Haut (6) angeordnet und mit monophasischen Stromimpulsen beaufschlagt wird.
